# EUROPEAN PATENT SPECIFICATION

(11) **EP 1 210 103 B1**
(45) Date of publication and mention of the grant of the patent: **22.12.2004**
(21) Application number: 00968999.3
(22) Date of filing: 18.08.2000
(51) Int. Cl.: A61K 38/19, A61K 38/24, A61K 38/29, A61P 25/00

(54) **THROMBOPOIETIN FOR TREATMENT OF NEUROLOGIC DAMAGE**
THROMBOPOIETIN ZUR BEHANDLUNG VON NEUROLOGISCHEN SCHÄDEN
THROMBOPOIETIN POUR LE TRAITEMENT DES LESIONS NEUROLOGIQUES

(30) Priority: 20.08.1999 US 150040 P; 07.02.2000 US 499198; 17.08.2000 US 642236
(43) Date of publication of application: 05.06.2002
(73) Proprietor: Schwartz, George R., Santa Fe, NM 87504 (US)
(72) Inventor: Schwartz, George R., Santa Fe, NM 87504 (US)
(74) Representative: Kupecz, Arpad
(86) International application number: PCT/US2000/040683
(87) International publication number: WO 2001/013936

(56) References cited:
- LI B ET AL: "Thrombopoietin and neurotrophins share a common domain." BLOOD. UNITED STATES 15 AUG 1995, vol. 86, no. 4, 15 August 1995 (1995-08-15), pages 1643-1644, XP002261467 ISSN: 0006-4971
- MELLOW A M ET AL: "A PILOT STUDY OF INTRAVENOUS THYROTROPIN-RELEASING HORMONE IN ALZHEIMER'S DISEASE" ANNALS OF THE NEW YORK ACADEMY OF SCIENCES, NEW YORK ACADEMY OF SCIENCES, NEW YORK, NY, US, 1989, pages 618-619, XP001087750 ISSN: 0077-8923
- DATABASE MEDLINE [Online] US NATIONAL LIBRARY OF MEDICINE, (BETHESDA, MD, USA) HEUER H. ET AL.: 'Expression of thyrotropin-releasing hormone receptor 2 (TRH-R2) in the central nervous system of rats', XP002935211 Database accession no. 11064370 & J. COMP. NEUROL. vol. 428, no. 2, 11 December 2000, pages 319 - 336
- DATABASE MEDLINE [Online] US NATIONAL LIBRARY OF MEDICINE, (BETHESDA, MD, USA) D'ALECY L.: 'Thyroid hormone in neural rescue', XP002935212 Database accession no. 9086579 & THYROID vol. 7, no. 1, 07 February 1997, pages 115 - 124
- DATABASE MEDLINE [Online] (COLUMBUS, OHIO, USA) NAKANISHI K. ET AL.: 'Thrombopoietin expression in normal and hypobaric hypoxia-induced thrombocytopenic rats', XP002935213 Retrieved from STN Database accession no. 1999304855 & LABORATORY INVESTIGATION vol. 79, no. 6, June 1999, pages 679 - 688

## Description

### BACKGROUND OF THE INVENTION

This PCT application claims priority of U.S. Patent Application Serial No. 09/642,236 entitled "Method of Enhancement of Neurologic Recovery in Human Nervous System Damage by Use of Pharmaceutical Thrombopoietin", to George R. Schwartz, filed on August 17, 2000, which is a continuation-in-part application of U.S. Patent Application Serial No. 09/587,552 entitled "Method of Enhancement of Neurologic Recovery in Human Nervous System Damage by Use of Pharmaceutical Thrombopoietin", to George R. Schwartz, filed on June 5, 2000, which in tum is a continuation-in-part of U.S. Patent Application Serial No. 09/499,198, entitled "Method of Enhancement of Neurologic Recovery in Human Nervous System Damage by Use of Pharmaceutical Thrombopoietin," to George R. Schwartz, filed February 7, 2000, now abandoned, and which claimed the benefit of U.S. Provisional Application Serial No. 60/150,040 , entitled "Method of Enhancement of Neurologic Recovery in Human Nervous System Damage by Use of Pharmaceutical Thrombopoietin", to George R. Schwartz, filed August 20, 1999.

### Field of the Invention (Technical Field):

This invention relates to the use of thrombopoietin for the manufacture of medicament for treatment of human neurologic damage, and in particular for increased regeneration and repair of damaged neurons and nerve axon myelin coatings and nerve cell regair.

### DESCRIPTION OF THE PREFERRED EMBODIMENTS (BEST MODES FOR CARRYING OUT THE INVENTION)

Demyelination occurs when the myelin coating around nerve axons degenerates resulting in a defect in the ability to transmit nerve impulses. For example, multiple sclerosis is a disease of unknown cause in which degeneration occurs in the myelin sheath surrounding the nerves. This demyelination is also found in many other diseases such as transverse myelitis. Demyelination also occurs after trauma to the brain or spinal cord, after a stroke, in neurodegenerative diseases such as amyotropic lateral sclerosis and Alzheimer's disease, as well as in viral diseases including AIDS.

A cell type in the nervous system called the oligodendroglia is intimately involved in myelin regeneration, repair and maintenance of the nerve cells. Repair occurs by the repetitive wrapping of the plasma membranes of the oligodendroglia cells around damaged nerve cells and offers continuing metabolic nerve cell support. In the art, it has been established that for 0-2A progenitor cells that produce oligodendroglia cells proliferation is induced in culture by type-1 astrocytes. A recognized mitogen for 0-2A progenitor cells is platelet-derived growth factor (PDGF), and PDGF is a potent mitogen for 0-2A progenitor cells in vitro. Thus, laboratory experimentation has suggested that PDGF is crucial for the control of nerve cell repair and myelination in the nervous system.

It is also known in the art that the development of oligodendrocytes from precursor cells also includes an effector component which depends on thyroid hormone that stops cell division and initiates differentiation at the appropriate time.

Further, it is also known in the art that proteins generally referred to as thrombopoietins support biological activity that ultimately results in the production of platelets and other cells from the myeloid line, including markedly increasing PDGF production. Methods of preparation of thrombopoietin are disclosed in recent patents, for example, U.S. 5,795,569 issued to Amgen, Inc. and processes for producing them by recombinant genetic engineering techniques are also disclosed. Hence, the availability of thrombopoietins in pharmaceutically available quantities is to be expected in the near future.

### SUMMARY OF THE INVENTION

This invention relates to the use of thrombopoietin for the manufacture of medicament for treatment of degenerative neurologic diseases by administration of therapeutically effective amounts of thrombopoietin, to enhance the regeneration of neuron cells. A regulatory agent, such as thyroid hormone or thyrotropin, may also be included as a regulator of cell division and differentiation. The use may be used with humans and also with other mammals with neurologic damage.

The thrombopoietin may be orally ingested by the patient, or may be administered by intravenous, intramuscular or intrathecal injection.

The use can further include the step of administering thyroid hormone to the patient. The thyroid hormone may be orally ingested by the patient, or may be administered by intravenous, intramuscular or intrathecal injection. The thyroid hormone may include thyroid hormone extract or synthetic thyroid hormone.

The use can also further include the step of stimulating human thyroid production by administering thyrotropin to the patient. The thyrotropin may be orally ingested by the patient, or may be administered by intravenous, intramuscular or intrathecal injection.

The thrombopoietin may be isolated from a mammal, made by recombinant means, or made by synthetic means. It may be human thrombopoietin, a fragment of human thrombopoietin, or a variant polypeptide of human thrombopoietin. The therapeutically effective amount of thrombopoietin ranges from about 1.0 to about 100 µg/kg body weight per day.

In the use of this invention, the thyroid hormone can be co-administered to the patient with the thrombopoietin. In an alternative method, the thyrotropin can be co-administered to the patient with the thrombopoietin.

The invention further consists of a pharmaceutical composition for treatment of neurologic damage in a mammal, comprising thrombopoietin and thyroid hormone. The composition can contain between about 0.07 to about 10 mg of thrombopoietin per dose unit. The composition may be formulated such that it contains between one and three times as much thyroid hormone as thrombopoietin. In this composition, the thrombopoietin may be isolated from a mammal, made by recombinant means, or made by synthetic means. The thrombopoietin may be human thrombopoietin, a fragment of human thrombopoietin, or a variant polypeptide of human thrombopoietin. The thyroid hormone may be thyroid hormone extract or synthetic thyroid hormone.

The invention further consists of a pharmaceutical composition for treatment of neurologic damage in a mammal, comprising thrombopoietin and thyrotropin. The composition can contain between 0.07 to 800 mg of thrombopoietin per dose unit. In this composition, the thrombopoietin may be isolated from a mammal, made by recombinant means, or made by synthetic means. The thrombopoietin may be human thrombopoietin, a fragment of human thrombopoietin, or a variant polypeptide of human thrombopoietin.

A primary object of the present invention is to provide a use and formulation for neuron or myelin regeneration and maintenance in the nervous system.

Another object of the present invention is to provide a use for neuron and myelin regeneration and maintenance using endogenous platelet-derived growth factor (PDGF), production of which is induced by administration of a therapeutically effective amount of thrombopoietin (TPO).

Another object of the invention is to provide a use for treatment of neurological disorders including administration of TPO and co-administration or sequential administration of a thyroid hormone, or thyrotropin.

Other objects, advantages and novel features, and further scope of applicability of the present invention will be set forth in part in the detailed description to follow, and in part will become apparent to those skilled in the art upon examination of the following, or may be learned by practice of the invention. The objects and advantages of the invention may be realized and attained by means of the instrumentalities and combinations particularly pointed out in the appended claims.

### DESCRIPTION OF THE PREFERRED EMBODIMENTS (BEST MODES FOR CARRYING OUT THE INVENTION)

The present invention discloses the use of thrombopoietin for the manufacture of medicament for treatment of peripheral and central neurological diseases by the administration to patients of thrombopoietin. The nervous system disorders, include diseases such as transverse myelitis, multiple sclerosis, demyelination occurring after trauma to the brain or spinal cord, as well as stroke, degenerative diseases such as Alzheimer's and amyotrophic lateral sclerosis, and viral diseases such as AIDS, as well as peripheral nerve injury. Thrombopoietin serves to stimulate the production of oligodendroglia and other glial cells in the repair of damaged neurons and nerve axons incident to such diseases.

Thrombopoietin may be administered as a liquid or solid in the form of oral tablets, intravenous injection, intrathecal injection or intramuscular injection, depending upon the course of therapy followed.

In a second embodiment of the invention, a regulatory agent is used, which is thyroid hormone in the form of thyroid extract, stimulation of human production by thryrotropin (thyroid-stimulating hormone), or synthetic thyroid hormone combined with thrombopoietin to regulate cell division and oligodendroglia production.

Prior to describing the present invention in detail, the following definitions are given:

Thrombopoietin: Includes TPO produced by any means, including TPO isolated from a mammal, made by recombinant means and made by synthetic means. TPO may be human TPO, a fragment of human TPO, a variant polypeptide of TPO, a chimeric polypeptide of any of the foregoing and the like. The TPO may further be modified, and may be pegylated, glycosolated and the like. The TPO may further be present in a formulation including one or more carriers or excipients. Various forms and formulations of TPO are described in, among others, U.S. Patent Nos. 5,795,569; 5,879,673; and 5,989,537.

Regulatory agent: Regulatory agents are thyroid hormone and thyrotropin. The effort of these regulatory agents are described generally in Rodriguez-Pena A: Oligodendrocyte development and thyroid hormone. *J. Neurobiol* 1999, Sep. 15;40(4):497-512; Ahlgren SC, Wallace H, Bishop J, Neophytou C, Raff MC: Effects of thyroid hormone on embryonic oligodendrocyte precursor cell development in vivo and in vitro. *Mol Cell Neurosci* 1997;9(5/6);420-32; Gao FB, Apperly J, Raff M: Cell-intrinsic timers and thyroid hormone regulate the probability of cell-cycle withdrawal and differentiation of oligodendrocyte precursor cells. *Dev Biol* 1998 May 1;197(1):54-66; Ahlgren SC, Wallace H, Bishop J, Neophytou C, Raff MC: Effects of thyroid hormone on embryonic oligodendrocyte precursor cell development in vivo and in vitro. *Mol Cell Neurosci* 1997;9(5/6);420-32; and Durand B, Raff M: A cell-intrinsic timer that operates during oligodendrocyte development. Bioessays 2000 Jan; 22(1):64-71. The thyroid hormone, thyrotropin may be isolated from a mammal, made by synthetic means, made by recombinant means, or made by any means known in the art. The regulatory agent may further be present in a formulation induding one or more carriers or excipients.

The uses and compositions of this invention may be used in the treatment of any of a variety of conditions resulting in neurological damage, including peripheral and central nervous system (CNS) injury, disease or disorders. CNS disorders encompass numerous afflictions such as neurodegenerative diseases (e.g. Alzheimer's and Parkinson's), acute brain injury (e.g. stroke, head injury, cerebral palsy) and a large number of CNS dysfunctions (e.g. depression, epilepsy, schizophrenia). In recent years neurodegenerative disease has become an important concern due to the expanding elderly population which is at greatest risk for these disorders. These diseases include Alzheimer's disease, multiple sclerosis (MS), Huntington's disease, amyotrophic lateral sclerosis, and Parkinson's disease. CNS disorders also encompass CNS trauma, such as results from stroke, epilepsy, traumatic injury and the like. Further, many viral diseases, including AIDS, result in CNS disorders.

TPO may be formulated as set forth above, and administered by any art conventional means. A therapeutically effective amount of TPO is administered. resulting in PDGF expression. Relatively low dosages may be employed, from about 1.0 to about 100 µg/kg body weight of the patient, and preferably about 5 to about 10 µg/kg body weight.

The TPO can be administered through various routes including via the nose or lung, cutaneously, subcutaneously, intrathecally, and preferably intravenously. Depending upon the route of administration, the TPO is preferably administered in combination with an appropriate pharmaceutically acceptable carrier or excipient. When administered systemically, the therapeutic composition should be pyrogen-free and in a parenterally acceptable solution. These conditions are generally well known and accepted to those of skill in the appropriate art.

Briefly, dosage formulations of the materials of the present invention are prepared for storage or administration by mixing the compound having the desired degree of purity with physiologically acceptable carriers, excipients and/or stabilizers. Such materials may include buffers such as phosphate, citrate, acetate and other organic acid salts antioxidants such as ascorbic acid; low molecular weight peptides such as polyarginine, proteins such as serum albumen, gelatin or immunogloulins; hydrophilic polymers such as polyvinylpyrrolidinone; amino acids such as glycine, glutamic acid, aspartic acid or arginine; monosaccharides, disaccharides and other carbohydrates including cellulose or its derivatives, glucose, mannose or dextrins; chelating agents such as EDTA; sugar alcohol such as mannitol or sorbitol; counter-ions such as sodium and/or non-ionic surfactants such as Tween, Pluronics or polyethylineglycol. The TPO may be administered as the free acid or base form or as a pharmaceutically acceptable salt.

The TPO may be administered in sustained-release formulations, including polymeric substrates, hydrogels, liposomes and the like.

The regulatory agent may similarly be formulated as set forth above, and administered by any art conventional means. In the case of thyroid hormone, therapeutically effective amounts may be employed; resulting in a decrease in cellular division of oligodendrocytes and further differentiation into functional neuronal repair cells.

Any of a variety of thyroid hormones may be employed as a regulatory agent, including but not limited to purified thyroid, levothyroxine, liothyronine, and thyroglobulin. These are available in oral tablet formulations, and in the case of levothyroxine, additionally as an injectable parenteral. In usual oral dosage forms, from about 0.10 to 0.125 mg per day of levothyroxine is administered, for liothyronine sodium from about 25 to 50 µg per day is administered, for thyroglobulin from about 32 to 160 µg per day is administered, and for dessicated thyroid from about 15 to 120 mg per day is administered. Combinations of the foregoing may also be administered, such as liotrix, a combination of levothyroxine and liothyronine. Injectable levothyroxine, from about 50 to 200 µg per day, may be injected into a muscle or into a vein.

Thyrotropin, also called thyroid-stimulating hormone, may alternatively be employed to increase endogenous thyroid production. Thyrotropin is available as an injectable parenteral. Thyrotropin may be produced by any means known in the art, including as a recombinant form of human thyroid-stimulating hormone. Sufficient thyrotropin is administered to increase thyroid levels to that desired, with monitoring as required by any of a variety of available thyroid assays.

In one embodiment, a patient with a CNS disorder is administered from 1 to 50 µg/kg body weight of TPO by intravenous injection, with such injections occurring every 2 days for a period of at least 8 days. At a suitable time, such as 10 days after the initial injection of TPO, administration of thyroid hormone is initiated. The thyroid hormone administration, for example a Synthroid® preparation (levothyroxine), is at a rate of about 2 to 4 µg/kg body weight per day, and is continued for a suitable period, such as 21 days.

Multiple courses of therapy may be undertaken, such that TPO and thyroid hormone administration are alternated. There may also be periods of time between courses of therapy in which periods may be fixed or may be related to the onset of symptoms of CNS disorder.

Thrombopoietin and regulatory agent may be co-administered; as part of a single formulation. Thus, a treatment regimen may be employed in which both TPO and thyroid hormone are co-administered. Alternatively, TPO may be initially administered without thyroid hormone and after a suitable period administration of a formulation including TPO and thyroid hormone or thyrotropin is initiated.

The invention is further illustrated by the following non-limiting examples.

### Example 1

B6SJL-TgN(SOD1-G93A)1Gur strain mice were obtained from The Jackson Laboratory, Bar Harbor, ME. The transgene in these mice carries a high copy number of a mutant allele human SOD1 containing the Gly93 ->Ala (G93A) substitution (often referred to as G1 H). Mice progressively become paralyzed in one or more limbs, with paralysis due to loss of motor neurons from the spinal cord. The mice are generally described in Gurney ME, Pu H, Chiu AY, Dal Canto MC, Polchow CY, Alexander DD, Caliendo J, Hentati A, Kwon YW, Deng HX, Chen W. Zhai P, Sufit RL, Siddique T: Motor neuron degeneration in mice that express a human Cu, Zn superoxide dismutase mutation. *Science* 264:1772-1775 (1994), and use of these mice as a model of familial amyotrophic lateral sclerosis is described in Chiu AY, Zhai P, Dal Canto MC, Peters TM, Kwon YW, Prattis SM, Gurney ME: Age-dependent penetrance of disease in a transgenic mouse model of familial amyotrophic lateral sclerosis. *Mol Cell Neurosci* 6:349-362 (1995).

At 105 days following birth, mouse A began manifesting objective symptoms of limb paralysis. A protocol as described in Table 1 was followed.

**Table 1**

| **Days Following Birth** | **Administration** |
|---|---|
| 107 | 0.5 cc i.p. of a solution containing 1 µg/cc of thrombopoietin |
| 110 | 0.5 cc i.p. of a solution containing 1 µg/cc of thrombopoietin |
| 113 | 0.5 cc i.p. of a solution containing 1 µg/cc of thrombopoietin |
| 117 | 0.5 cc i.p. of a solution containing 1 µg/cc of thrombopoietin and 0.25 cc i.p. of a solution containing 500 µg/cc of thyrotropin releasing hormone |
| 119 | 0.5 cc i.p. of a solution containing 1 µg/cc of thrombopoietin |
| 121 | Synthroïd®, 0.1 mg in 2 ounces of drinking water, ad libitum |

The thrombopoietin was obtained from Rand D. Systems, Inc. (Minneapolis, MN) as carrier-free recombinant thrombopoietin and the thryrotropin was obtained from Ferring Pharmaceuticals; both were administered by intraperitoneal (i.p.) injection. Synthroid®, a form of levothyroxine, was obtained from Flint Pharmaceuticals in 100 µg tablets.

At day 111, marked improvement was observed, with elimination of shaking and limpness and improved muscle tone. At day 113, shaking resumed with observed early weakness developing. By day 115 marked hind limb weakness was observed. By day 118, decreased weakness was observed, although paresis remained in the left hind quarters. On day 120, increased mobility was observed. On day 122, marked improvement in front limbs was observed. Recurrence and increasing front limb weakness was observed on day 124. The mouse died at day 126.

### Example 2

A litter mate of mouse A, referred to as mouse B, was administered a protocol as described in **Table 2**, with administration commencing prior to the onset of any objective symptoms.

**Table 2**

| **Days Following Birth** | **Administration** |
|---|---|
| 87 | 0.5 cc i.p. of a solution containing 1 µg/cc of thrombopoietin |
| 91 | 0.5 cc i.p. of a solution containing 1 µg/cc of thrombopoietin |
| 93 | 0.6 cc i.p. of a solution containing 1 µg/cc of thrombopoietin |
| 95 | 0.5 cc i.p. of a solution containing 1 µg/cc of thrombopoietin |
| 105 | Synthroid®, 0.1 mg in 4 ounces of drinking water, ad libitum through remaining life |
| 126 | 0.5 cc i.p. of a solution containing 1 µg/cc of thrombopoietin |

With this protocol, no objective signs were observed until day 121, when slight posterior weakness was noted. This was 16 days following onset of objective signs of illness in the litter mate mouse A. At day 129, posterior limb paralysis was observed, with the fore limbs remaining functional but some weakness observed in the left front. The mouse died on day 132.

### Example 3

A B6SJL-TgN(SOD1-G93A)1Gur strain mouse as described in Example 1 was obtained, referred to as mouse C. Mouse C was administered a protocol as described in **Table 3**, with administration commencing prior to the onset of any objective symptoms.

**Table 3**

| **Days Following Birth** | **Administration** |
|---|---|
| 80 | 0.5 cc i.p. of a solution containing 1 µg/cc of thrombopoietin |
| 84 | 0.5 cc i.p. of a solution containing 1 µg/cc of thrombopoietin |
| 86 | 0.6 cc i.p. of a solution containing 1 µg/cc of thrombopoietin |
| 88 | 0.5 cc i.p. of a solution containing 1 µg/cc of thrombopoietin |
| 92 | Synthroid®, 0.1 mg in 4 ounces of drinking water, ad libitum through remaining life |

With this protocol, no objective signs of illness were observed through day 110, more than two standard deviations from the expected onset of sickness (91 ± 14 days).

The preceding examples can be repeated with similar success by substituting the generically or specifically described reactants and/or operating conditions of this invention for those used in the preceding examples.

Although the invention has been described in detail with particular reference to these preferred embodiments, other embodiments can achieve the same results. Variations and modifications of the present invention will be obvious to those skilled in the art and it is intended to cover in the appended claims all such modifications and equivalents.

## Claims

1. A pharmaceutical composition for treatment of neurologic damage in a mammal, comprising thrombopoietin and thyroid hormone.

2. A pharmaceutical composition for treatment of neurologic damage in a mammal, comprising thrombopoietin and thyrotropin.

3. The compositions of claim 1 or 2, comprising between 0.07 to 10 mg of thrombopoietin per dose unit.

4. The compositions of claim 1 or 2 wherein the thrombopoietin is a thrombopoietin isolated from a mammal, a thrombopoietin made by recombinant means,.or a thrombopoietin made by synthetic means.

5. The compositions of claim 1 or 2 wherein the thrombopoietin is human thrombopoietin, a fragment of human thrombopoietin, or a variant polypeptide of human thrombopoietin.

6. The composition of claim 3 wherein the composition contains between one and three times the usual dose for thyroid hormone as for thrombopoietin.

7. The use of thrombopoietin for the manufacture of medicament for treatment of neurologic damage in a mammal by administration of therapeutically effective amounts of thrombopoietin to the mammal.

8. The use of claim 7 wherein thyroid hormone is to be administered to the mammal.

9. The use of claim 7 wherein thyroid production in the mammal is to be stimutated by administering thyrotropin.

10. The use of claim 7 wherein administration of therapeutically effective amounts of thrombopoietin comprises oral administration, intravenous injection, intramuscular injection, or intrathecal injection.

11. The use of claim 8 wherein administration of thyroid hormone comprises oral administration, intravenous injection, intramuscular injection, or intrathecal injection.

12. The use of claim 8 wherein thyroid hormone comprises thyroid hormone extract or synthetic thyroid hormone.

13. The use of claim 9 wherein administration of thyrotropin comprises oral administration, intravenous injection, intramuscular injection, or intrathecal injection.

14. The use of claim 7 wherein the thrombopoietin is a thrombopoietin isolated from a mammal, a thrombopoietin made by recombinant means, or a thrombopoietin made by synthetic means.

15. The use of claim 7 wherein the thrombopoietin is human thrombopoietin, a fragment of human thrombopoietin, or a variant polypeptide of human thrombopoietin.

16. The use of claim 7 wherein the therapeutically effective amount ranges from 1.0 to 100 µg/kg body weight per day.

17. The use of claim 8 wherein the thyroid hormone is to be co-administered to the mammal with the thrombopoietin.

18. The use of claim 9 wherein the thyrotropin is to be co-administered to the mammal with the thrombopoietin.

## Patentansprüche

1. Pharmazeutische Zusammensetzung für die Behandlung von neurologischen Schäden in einem Säugetier, aufweisend Thrombopoietin und Thyroidhormon.

2. Pharmazeutische Zusammensetzung für die Behandlung von neurologischen Schäden in einem Säugetier, aufweisend Thrombopoietin und Thyrotropin.

3. Zusammensetzung gemäß Anspruch 1 oder 2, aufweisend zwischen 0.07 und 10 mg Thrombopoietin pro Dosiseinheit.

4. Zusammensetzung gemäß Anspruch 1 oder 2, wobei das Thrombopoietin ein aus einem Säugetier isoliertes Thrombopoietin, ein mittels Rekombination hergestelltes Thrombopoietin oder ein auf synthetischem Wege hergestelltes Thrombopoietin ist.

5. Zusammensetzung gemäß Anspruch 1 oder 2, wobei das Thrombopoietin humanes Thrombopoietin, ein Fragment von humanem Thrombopoietin oder eine Polypeptid-Variante des humanen Thrombopoietin ist.

6. Zusammensetzung gemäß Anspruch 3, wobei die Zusammensetzung zwischen dem ein- und dem dreifachen der üblichen Dosis für Thyroidhormon als für Thrombopoietin enthält.

7. Verwendung von Thrombopoietin für die Herstellung eines Medikaments für die Behandlung von neurologischen Schäden in einem Säugetier durch Verabreichung von therapeutisch wirksamen Dosen von Thrombopoietin an das Säugetier.

8. Verwendung gemäß Anspruch 7, wobei dem Säugetier Thyroidhormon verabreicht wird.

9. Verwendung gemäß Anspruch 7, wobei die Thyroid-Produktion des Säugetiers durch die Verabreichung von Thyrotropin angeregt wird.

10. Verwendung gemäß Anspruch 7, wobei die Verbreichung von therapeutisch wirksamen Dosen von Thrombopoietin orale Verbreichung, intravenöse Injektion, intramuskuläre Injektion oder intrathekale Injektion umfaßt.

11. Verwendung gemäß Anspruch 8, wobei die Verbreichung von Thyroidhormon orale Verbreichung, intravenöse Injektion, intramuskuläre Injektion oder intrathekale Injektion umfaßt.

12. Verwendung gemäß Anspruch 8, wobei Thyroidhormon Thyroidhormon-Extrakt oder synthetisches Thyroidhormon umfaßt.

13. Verwendung gemäß Anspruch 9, wobei Verabreichung von. Thyrotropin orale Verbreichung, intravenöse Injektion, intramuskuläre Injektion oder intrathekale Injektion umfaßt.

14. Verwendung gemäß Anspruch 7, wobei das Thrombopoietin ein aus einem Säugetier isoliertes Thrombopoietin, ein mittels Rekombination hergestelltes Thrombopoietin oder ein auf synthetischem Wege hergestelltem Thrombopoietin ist.

15. Verwendung gemäß Anspruch 7, wobei das Thrombopoietin humanes Thrombopoietin, ein Fragment von humanem Thrombopoietin oder eine Polypeptid-Variante des humanen Thrombopoietin ist.

16. Verwendung gemäß Anspruch 7, wobei die therapeutisch wirksame Dosis im Bereich zwischen 1 und 100 µg/kg Körpergewicht pro Tag liegt.

17. Verwendung gemäß Anspruch 8, wobei das Thyroidhormon dem Säugetier zusammen mit dem Thrombopoietin verabreicht wird.

18. Verwendung gemäß Anspruch 9, wobei das Thyrotropin dem Säugetier zusammen mit dem Thrombopoietin verabreicht wird.

## Revendications

1. Composition pharmaceutique destinée au tracement de lésions neurologiques chez un mammifère, comprenant de la thrombopoïétine et de l'hormone thyroïdienne.

2. Composition pharmaceutique destinée au traitement de lésions neurologiques chez un mammifère, comprenant de la thrombopoïétine et de la thyréostimuline.

3. Compositions selon la revendication 1 ou 2, comprenant de 0,07 à 10 mg de thrombopoïétine par unité posologique.

4. Compositions selon la revendication 1 ou 2, dans lesquelles la thrombopoïétine est une thrombopoïétine isolée d'un mammifére, une thrombopoïétine faite par des moyens de recombinaison, ou une thrombopoïetine faite par des moyens de synthèse.

5. Compositions selon la revendication 1 ou 2, dans lesquelles la thrombopoïétine est la thrombopolétine humaine, un fragment de thrombopaïétine humaine, ou un polypeptide variant de thrombopoiétine humaine,

6. Composition selon la revendication 3, dans laquelle la composition contient entre une et trois fois la dose habituelle d'hormone thyroïdienne par rapport à la thrombopoïétine.

7. Utilisation de thrombopoïétine pour la fabrication d'un médicament destiné au traitement de lésions neurologiques chez un mammifère par l'administration de quantités efficaces sur le plan thérapeutique de thrombopoïétine au mammifère.

8. Utilisation selon la revendication 7, dans laquelle l'hormone thyroïdienne doit être administrée au mammifère.

9. Utilisation selon la revendication 7, dans laquelle la production thyroïdienne chez le mammifère doit être stimulée par l'administration de thyréostimuline.

10. Utilisation selon la revendication 7, dans laquelle l'administration de quantités de thrombopoïétine efficaces sur le plan thérapeutique comprend l'administration par voie orale, l'injection intraveineuse, l'injection intramusculaite, ou l'injection intrathécale.

11. Utilisation selon la revendication 8, dans laquelle l'administration d'hormone thyroïdienne comprend l'administration par voie orale, l'injection intraveineuse, l'injection intramusculaire, ou l'injection intrathécale.

12. Utilisation selon la. revendication 8, dans laquelle l'hormone thyroïdienne comprend l'extrait d'hormone thyroïdienne ou l'hormone thyroïdienne de synthèse.

13. Utilisation selon la revendication 9. dans laquelle l'administration de thyréostimuline comprend l'administration par voie orale, l'injection intraveineuse, l'injection intramusculaire, ou l'injection intrathécale.

14. Utilisation selon la revendication 7, dans laquelle la thrombopozétine est une thrombopoïétine isolés d'un mammifère, une thrombopoïétine faite par des moyens de recombinaison, ou une thrombopoïétine faite par des moyens de synthèse.

15. Utilisation selon la revendication 7, dans laquelle la thrombopoïétine est la thrombopoïétine humaine, un fragment de la thrombopoïétine humaine, ou un polypeptide variant de la thrombopoïétine humaine.

16. Utilisation selon la revendication 7, dans laquelle la quantité efficace sur le plan thérapeutique se trouve dans la plage de 1,0 à 100 µg/kg de poids corporel par jour.

17. Utilisation selon la revendication 8, dans laquelle l'hormone thyroïdienne doit être co-administrée au mammifère avec la thrombopoïétine.

18. Utilisation selon la revendication 9, dans laquelle la thyréostimuline doit être co-administrée au mammifère avec la thrombopoïétine.
